Europäisches Patentamt

European Patent Office

Office européen des brevets

(11) Publication number: **0 328 099**
**A1**

# (12) EUROPEAN PATENT APPLICATION

(21) Application number: **89102235.2**

(22) Date of filing: **09.02.89**

(51) Int. Cl.4: **A61K 7/42 , A61K 7/44**

(30) Priority: **11.02.88 US 155041**

(43) Date of publication of application:
**16.08.89 Bulletin 89/33**

(84) Designated Contracting States:
**DE FR GB**

(71) Applicant: **ESTEE LAUDER INC.**
**767 Fifth Avenue**
**New York New York 10153(US)**

(72) Inventor: **Pelliccione, Nicholas J.**
**665 East 17th Street**
**Brooklyn New York 11230(US)**
Inventor: **The other inventor has agreed to**
**waive his entitlement to designation**

(74) Representative: **Vossius & Partner**
**Siebertstrasse 4 P.O. Box 86 07 67**
**D-8000 München 86(DE)**

(54) Tanning compositions and their use.

(57) Compositions and methods for the tanning of the skin and the protection of the skin from the harmful effects of ultraviolet light. A preferred composition of the invention comprises N-acetyl L-tyrosine, a vasodilator, and a carrier.

EP 0 328 099 A1

## TANNING COMPOSITIONS AND THEIR USE

This invention relates to tanning compositions and methods for using such compositions. The tanning compositions of the invention, when topically applied to the skin, enhance (i.e., speed up) the normal biological processes of the epidermis that result in the tanning of the skin. In addition, the compositions of the invention protect the skin from the harmful effects of ultraviolet light.

Exposure of the human skin to ultraviolet light rays, produced naturally by the sun or artificially by sources such as sun lamps, can have both desired and harmful effects on the skin. Harmful effects, resulting mainly from exposure to ultraviolet rays with wavelengths from 200 nm to 320 nm (UVB radiation), include erythema ("sunburn") and skin cancers, such as melanomas ("tumors"). Desired effects, resulting primarily from exposure to the ultraviolet wavelengths of from about 320 nm up to about 400 nm (UVA radiation), include the aesthetically pleasing tanning of the skin.

Compositions called "sunscreens" are known that protect the skin from the effects of harmful ultraviolet rays. Such sunscreens are usually incorporated in a cream, lotion, or gel vehicle.

Some sunscreens take the form of opaque formulations that function primarily by reflecting and scattering radiation. Other sunscreens contain ultraviolet-absorbing chemicals that absorb and filter out ultraviolet rays in the harmful range.

Compositions are also known that use chemical agents, such as dihydroxyacetone, erythrulose, and glycerose, to dye the skin, thereby giving the appearance of a sun tan. Such compositions have enjoyed little, if any, commercial success because of concerns with their safety and the relatively poor results obtained with their use.

Other tanning compositions are known that use natural substances to promote photo-pigmentation and photo-protection of the skin during exposure to ultraviolet rays. Such compositions comprise, for example, an amino acid or derivative thereof used in combination with L-tyrosine. See U.S. Patent 4,419,343 (the '343 patent). Compositions of the type described in the '343 patent, however, have their limits. For example, tanning compositions having a higher degree of solubility and a greater ability to penetrate through the outer layers of the skin than compositions of the type described in the '343 patent are desirable.

An object of this invention is to provide an improved composition for the tanning of the skin.

Another object of this invention is to provide a tanning composition that protects the skin from the harmful effects of ultraviolet radiation.

Still another object of the invention is to provide an improved method for tanning the skin while simultaneously protecting the skin from adverse effects caused by the exposure of the skin to ultraviolet radiation.

The foregoing and other desirable advantages are obtained in one embodiment of the invention by a composition that comprises N-acetyl L-tyrosine in a carrier. While not being bound by any particular theory, we believe that the ester bond between the N-acetyl group and the L-tyrosine enhances the ability of the composition's active ingredient(s) to enter the melanocytes found in the human skin, where a chemical reaction occurs that results in the production of melanin. Not only does this production of melanin have a desirable tanning effect, but it also enhances the ability of the skin to protect itself from the adverse effects of ultraviolet radiation. This is because the increase in the skin's melanin content not only darkens the skin, but also enhances the ability of the skin to filter harmful ultraviolet rays, thereby minimizing the damage caused by those rays.

The composition of the invention may also include a second tanning agent in addition to N-acetyl L-tyrosine. The additional tanning agent serves as a supplemental source of the tyrosine resulting in the production of melanin. We believe that the penetration of the second tanning agent into the skin is enhanced by the presence of the N-acetyl L-tyrosine in the composition, thereby resulting in a synergistic effect when the two tanning agents are used in combination.

The second tanning agent comprising a source of tyrosine suitable for use in our invention can comprise any material, other than N-acetyl L-tyrosine, that when applied to the skin supplies a source of tyrosine. Suitable second tanning agents for use in this invention include L-tyrosine, histidine, urocanic acid, and glutamic acid. Preferably, the composition of the invention comprises about 0.5 to 4.0% by weight of the second tanning agent.

In another embodiment of the invention, the composition of the invention comprises a vasodilator, i.e., a material that increases the micro-circulation or blood flow in the skin. We believe that the additional oxygen provided as a result of the vasodilator's causing increased blood flow results in a larger amount of melanin being produced by the composition of the invention.

While the vasodilator is preferably used in combination with N-acetyl L-tyrosine, we believe that the beneficial results that occur because of its use will occur when the vasodilator is used in combination with any source of tyrosine. Accordingly, the present invention, in a broad embodiment, is directed to a composition that comprises a source of tyrosine, a vasodilator, and a carrier.

As used herein, the term "carrier" shall include materials that are suitable for use in contact with the skin and which, when combined with the active ingredient in the composition of the invention, take a form that is suitable for application to the skin, e.g., the form of a liquid, gel, fluid, ointment, cream, lotion or the like.

The preferred carrier for use in this invention comprises an emulsion comprising oils in deionized water. The oils are selected so that, when combined with an emulsifier and the other components of the composition, a cohesive mixture of a texture suitable for application to the skin is obtained.

The compositions of the invention may be applied in effective amounts to the skin in any suitable manner. The amount applied and the frequency of application will, of course, vary depending upon the intensity of the source of ultraviolet radiation and the sensitivity of the individual using the composition to the effects of ultraviolet radiation.

The N-acetyl L-tyrosine of the present invention preferably comprises between 0.5 to 10.0% by weight of the composition of the invention. Suitable N-acetyl L-tyrosine may be obtained, in powdered form, from Ajinomoto, Inc., of Tokyo, Japan.

The composition preferably contains about 0.1 to 10.0% by weight of the vasodilator. The most preferred vasodilator for use in the present invention comprises the natural ingredients of butcher's broom and horse-chestnut extract. Such material is known by the tradename Peristem®, which is manufactured by Laboratories Serobiologique, Nancy, France. Other vasodilators acceptable for use in the invention include methylsalicylate, methylnicotinate, and tocopherol nicotinate.

Techniques commonly used in the cosmetic industry may be used to combine the ingredients of the composition of the present invention, particularly techniques in the industry that are used to manufacture oil and water emulsions. The water phase of the composition of the invention, which preferably comprises the tanning agent(s), preservatives, deionized water and a vasodilator (if used), are heated to 75° C and mixed until they are dissolved. Similarly, the various oils that comprise the oil phase of the invention are mixed and heated at 75° C in the presence of an emulsifier. The water and oil phases are then mixed to 75° C until well homogenized, after which they are cooled.

A base may also be included in the composition of the invention to neutralize the mixture. Any base commonly used to neutralize cosmetic compositions may be used, with the preferred base being triethanolamine.

To facilitate the formation of the oil and water emulsion, an emulsifier is added during the preparation of the oil phase. The emulsifier used may be selected from those commonly used in cosmetic emulsions, with the preferred emulsifier comprising glyceryl stearate and polyethylene glycol stearate. One such emulsifier, having the tradename Arlacel®, may be obtained from ICI Americas, Inc., in Wilmington, Delaware.

The carrier for use in the invention may also have incorporated therein a number of additives suitable for cosmetic or pharmaceutical use. For example, a fragrance may also be included to enhance the scent of the composition. Also, additional amounts of polyethylene glycol may be added to increase the viscosity of the composition of the invention.

The following example is presented for the sole purpose of further illustrating the present invention and is not to be taken as limiting thereto. Unless otherwise specified, all parts and percentages are by weight.

## EXAMPLE

An emulsion is prepared comprising a N-acetyl L-tyrosine, a second source of tyrosine, a vasodilator, and additional ingredients having the following composition:

| Components | % by weight |
|---|---|
| **Water Phase** | |
| N-acetyl L-tyrosine | 3.00 |
| Peristem® | 5.00 |
| triethanolamine 99 | 2.00 |
| methyl paraben | 0.30 |
| Germall 115 | 0.30 |
| deionized water | q.s. |
| **Oil Phase** | |
| sesame oil | 1.50 |
| stearic acid | 2.75 |
| cetyl alcohol | 0.80 |
| Arlacel® 165 | 3.65 |
| Robane | 1.00 |
| ceraphyl 50 | 53.00 |

The above composition may be made by combining the listed components in the manner described above.

**Claims**

1. A composition useful for the tanning of the skin comprising N-acetyl L-tyrosine and a carrier.

2. The composition of claim 1 wherein the N-acetyl L-tyrosine comprises about 0.5 to 10.0% by weight of the total composition.

3. The composition of claim 1 further comprising a second tanning agent comprising a second source of tyrosine.

4. The composition of claim 3 wherein the second tanning agent comprises from about 0.5 to 4.0% by weight of the total composition.

5. The composition of claim 4 wherein the N-acetyl L-tyrosine comprises about 0.5 to 10.0% by weight of the total composition.

6. A composition useful for the tanning of the skin comprising N-acetyl L-tyrosine, a vasodilator, and a carrier.

7. The composition of claim 6 further comprising a second tanning agent comprising a second source of tyrosine.

8. The composition of claim 6 wherein the vasodilator comprises from about 0.1 to 10.0% by weight of the total composition and is selected from the group consisting of (a) a mixture of butcher's broom and horsechestnut extract; (b) methylsalicylate; (c) methylnicotinate; (d) tocopherol nicotinate; and (e) mixtures thereof.

9. The composition of claim 6 wherein the N-acetyl L-tyrosine comprises about 0.5 to 10.0% by weight of the total composition and the vasodilator comprises about 0.1 to 10.0% by weight of the total composition.

10. A composition useful for the tanning of the skin comprising a vasodilator, a source of tyrosine and a carrier.

11. The composition of claim 10 where the source of tyrosine comprises about 0.5 to 10.0% by weight of the total composition, and the vasodilator comprises about 0.1 to 10.0% by weight of the total composition.

12. The composition of claim 10 wherein the vasodilator comprises from about 0.1 to 10.0% by weight of the total composition and is selected from the group consisting of (a) a mixture of butcher's broom and horsechestnut extract; (b) methylsalicylate; (c) methylnicotinate; (d) tocopherol nicotinate; and (e) mixtures thereof.

4

13. The composition of claim 10 wherein the vasodilator comprises from about 0.1 to 10.0% by weight of the total composition and is selected from the group consisting of (a) a mixture of butcher's broom and horsechestnut extract; (b) methylsalicylate; (c) methylnicotinate; (d) tocopherol nicotinate; and (d) mixtures thereof.

14. Use of the compositions according to any of claims 1 to 13 for the preparation of topically applicable skin tanning compositions.

## DOCUMENTS CONSIDERED TO BE RELEVANT

EP 89102235.2

| Category | Citation of document with indication, where appropriate, of relevant passages | Relevant to claim | CLASSIFICATION OF THE APPLICATION (Int. Cl.4) |
|---|---|---|---|
| P,X | GB - A - 2 198 042 (INDUCHEM A.G.) <br> * Examples 1,2 * | 1-14 | A 61 K 7/42 <br> A 61 K 7/44 |
| P,X | FR - A - 2 608 424 (C.CAZACOU) <br> * Totality * | 10-14 | |
| P,X | EP - A1 - 0 255 964 (CHEMISCH ADVIESBUREAU) <br> * Claim 1 * | 1-14 | |
| X | CH - A5 - 642 537 (UNI-CHEMIE AG) <br> * Totality * | 3,7, 10,11, 14 | |
| X | DE - A1 - 2 932 923 (H.RUBINSTEIN) <br> * Pages 3-6 * | 3,7, 10,14 | TECHNICAL FIELDS SEARCHED (Int. Cl.4) |
| X | EP - A1 - 0 010 483 (LABORATOIRES SEROBIOLOGIQUE S.A.) <br> * Claims 1,3 * | 3,7, 10,11, 14 | A 61 K 7/00 |
| A | US - A - 4 707 354 (D.GARLEN et al.) <br> * Totality * | 1-14 | |
| A | EP - A2 - 0 251 398 (THE PROCTER & GAMBLE COMPANY) <br> * Examples 8-14 * | 1-14 | |

The present search report has been drawn up for all claims

| Place of search | Date of completion of the search | Examiner |
|---|---|---|
| VIENNA | 12-05-1989 | IRMLER |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | P.H. LIST et al. "Hagers Handbuch der pharmazeutischen Praxis", vol. 4, 1973<br><br>SPRINGER, Berlin, Heidelberg, New York<br>pages 430-434<br><br>* Page 432 * | 1,8, 13,14 | |
| A | K. ROTHEMANN "Das große Rezeptbuch der Haut- und Körperpflegemittel" edition 2, 1956<br><br>DR. ALFRED HÜTHIG, Heidelberg<br><br>* Page 51 * | 1,8, 13,14 | |
| A | H. JANISTYN "Taschenbuch der modernen Parfumerie und Kosmetik", 1966<br><br>WISSENSCHAFTLICHE VERLAGSGESELL-SCHAFT, Stuttgart<br><br>* Page 344 * | 1,8, 13,14 | TECHNICAL FIELDS SEARCHED (Int. Cl.4) |
| A | FEY, OTTE "Wörterbuch der Kosmetik", 1985<br><br>WISSENSCHAFTLICHE VERLAGSGESELL-SCHAFT MBH, Stuttgart<br>page 168<br><br>* Methylsalycilat * | 1,8, 13,14 | |

The present search report has been drawn up for all claims

| Place of search | Date of completion of the search | Examiner |
|---|---|---|
| VIENNA | 12-05-1989 | IRMLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82